# EUROPEAN PATENT APPLICATION

(11) **EP 4 265 292 A1**
(43) Date of publication of application: **25.10.2023**
(21) Application number: 20966017.4
(22) Date of filing: 18.12.2020
(51) Int. Cl.: A61M 25/00, A61M 25/09

(54) **LONG MEDICAL INSTRUMENT**

(71) Applicant: Asahi Intecc Co., Ltd., Seto-shi, Aichi 489-0071 (JP)
(72) Inventor: NOGUCHI, Naoki, Seto-shi, Aichi 489-0071 (JP); TAKAHASHI, Hiroaki, Seto-shi, Aichi 489-0071 (JP); KAKUMU, Naoki, Seto-shi, Aichi 489-0071 (JP)
(74) Representative: Prinz & Partner mbB
(86) International application number: PCT/JP2020/047509
(87) International publication number: WO 2022/130631

(57) **Abstract**

Provided is an elongated medical device capable of improving visibility.

An elongated medical device 1 includes a first layer 11 having a hollow cylindrical shape and containing a fluororesin having an adhesive functional group (adhesive resin 111) and a first colorant 112, and a second layer 21 arranged in contact with an outer peripheral surface of the first layer 11 and having a predetermined colored region R formed of a second colorant 212 having a color different from a color of the first colorant 112, in which the predetermined colored region R is formed on a part of the outer peripheral surface of the first layer 11.

## Description

### TECHNICAL FIELD

The disclosed embodiments relate to an elongated medical device.

### BACKGROUND ART

For example, there are known medical devices such as guide wires, which have a visible marker for improving visibility in blood vessels via an endoscope that is inserted together with the medical devices (for example, see Patent Literature 1).

When using a medical device with the above technology, for example, a colored region may be provided to a specific site on an outermost layer and this colored region may be used as a clue to identify a position of the medical device inserted into a blood vessel.

### CITATION LIST

### Patent Literature

Patent Literature 1: WO 2010/018762A

### SUMMARY OF INVENTION

### TECHNICAL PROBLEM

The disclosed embodiments have been made in light of the above circumstances, and an object of the disclosed embodiments is to provide an elongated medical device capable of improving visibility.

### SOLUTION TO PROBLEM

The present disclosure includes some aspects described below.
(1) An elongated medical device including a first layer having a hollow cylindrical shape and containing a fluororesin having an adhesive functional group and a first colorant, and a second layer arranged in contact with an outer peripheral surface of the first layer and having a predetermined colored region formed of a second colorant having a color different from a color of the first colorant, in which the predetermined colored region is formed on a part of the outer peripheral surface of the first layer,
(2) The elongated medical device according to (1), in which the predetermined colored region is disposed in a transparent resin different from the fluororesin having the adhesive functional group,
(3) The elongated medical device according to (2), in which the transparent resin is at least one selected from a group consisting of polytetrafluoroethylene, tetrafluoroethylene-perfluoroalkylvinyl ether copolymer, tetrafluoroethylene-hexafluoropropylene copolymer, nylon, and urethane,
(4) The elongated medical device according to any one of (1) to (3), further including a metal core wire arranged in contact with an inner peripheral surface of the first layer,
(5) The elongated medical device according to any one of (1) to (3), further including: an adhesive intermediate layer having a hollow cylindrical shape and arranged in contact with the inner peripheral surface of the first layer; and a metal core wire arranged in contact with an inner peripheral surface of the intermediate layer,
(6) The elongated medical device according to any one of (1) to (3), further including a metal layer having a substantially hollow cylindrical shape and arranged in contact with the inner peripheral surface of the first layer, and
(7) The elongated medical device according to any one of (1) to (3), and (6), further including a sliding layer having a hollow cylindrical shape and disposed on an innermost side in a radial direction.

Note that, in this specification, the "fluororesin having an adhesive functional group" refers to a fluororesin having a functional group (adhesive functional group) that can participate in adhesion to an adjacent material such as a resin or a metal.

### ADVANTAGEOUS EFFECTS OF INVENTION

The disclosed embodiments make it possible to provide an elongated medical device capable of improving visibility.

### BRIEF DESCRIPTION OF DRAWINGS

FIG. 1 is a schematic longitudinal sectional view illustrating the first embodiment.
FIG. 2A is a schematic transverse sectional view taken along line IIA-IIA in FIG. 1.
FIG. 2B is a schematic transverse sectional view taken along line IIB-IIB in FIG. 1.
FIG. 3A is a schematic transverse sectional view illustrating a site where a colored region is formed in the second embodiment.
FIG. 3B is a schematic transverse sectional view illustrating a site where no colored region is formed in the second embodiment.
FIG. 4 is a schematic longitudinal sectional view illustrating the third embodiment.
FIG. 5 is a schematic longitudinal sectional view illustrating the fourth embodiment.

### DESCRIPTION OF EMBODIMENTS

Hereinafter, the first to fourth embodiments of the disclosed embodiments will be explained below with reference to the figures, but the disclosed embodiments are not limited to only the embodiments illustrated in the figures. Furthermore, the dimensions of the elongated medical device illustrated in each figure are indicated to facilitate the understanding of the contents of the implementation and do not correspond to the actual dimensions. Also, in each figure, the left side of the figure is the distal end side (farther side) to be inserted into the body, and the right side is the proximal end side (nearer side, hand side) to be operated by an operator such as a surgeon.

### [First Embodiment]

Patent Literature 1 describes a guide wire having a core wire, and a fluororesin disposed on an outer layer of the core wire. When the core wire is made of a metal in the guide wire having such a structure, it is necessary to dispose a binder layer as a base between the core wire and the fluororesin because adhesiveness between the core wire and the outer layer formed of the fluororesin deteriorates. When the binder layer is composed of a resin such as polyamideimide, a color of the binder layer as a base tends to assimilate with a color of a body tissue because a color of polyamideimide is close to a color of body tissues such as blood vessels. Thus, it is necessary to dispose a colored base layer on the outer periphery of the binder layer and a colored layer that serves as a visible marker on the outer periphery of the colored base layer, resulting in increase in an outer diameter of the guide wire.

An elongated medical device according to the present disclosure includes a first layer having a hollow cylindrical shape and containing a fluororesin having an adhesive functional group (hereinafter, referred to as "adhesive resin") and a first colorant, and a second layer arranged in contact with an outer peripheral surface of the first layer and having a predetermined colored region formed of a second colorant having a color different from a color of the first colorant, in which the predetermined colored region is formed on a part of the outer peripheral surface of the first layer.

In the configuration of the elongated medical device according to the present disclosure, the aforementioned binder layer and colored base layer can be composed of one layer (the first layer containing the first colorant), for example, to improve visibility while suppressing the increase in the outer diameter of the guide wire.

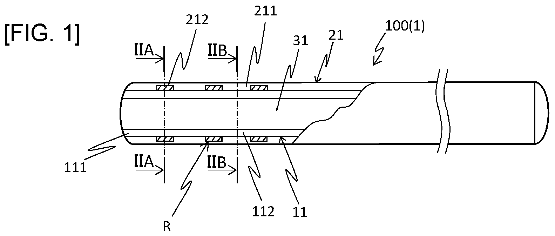

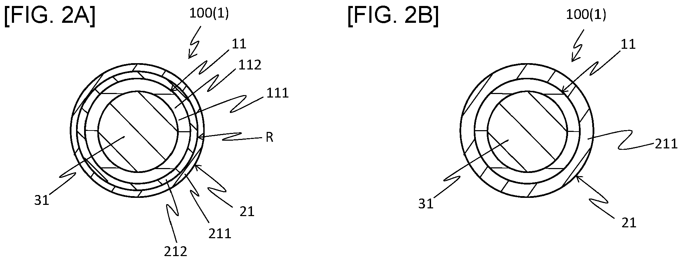

FIG. 1 and FIG. 2 are schematic diagrams illustrating the first embodiment. As schematically illustrated in FIG. 1, FIG. 2A, and FIG. 2B, an elongated medical device 1 is composed of a core wire 31, a first layer 11, and a second layer 21. In the first embodiment, as the elongated medical device 1, a guide wire 100 is described as an example.

The core wire 31 is a metal member that serves as a core material for the elongated medical device 1. The core wire 31 is arranged in contact with an inner peripheral surface of the first layer 11. Specifically, the core wire 31 can be composed of e.g. an elongated wire made of one solid metal material.

As a material constituting the core wire 31, for example, stainless steel such as SUS304, a superelastic alloy such as a Ni-Ti alloy, and the like can be adopted from the viewpoint of improving flexibility of the guide wire 100 and providing antithrombogenicity and biocompatibility.

As the dimension of the core wire 31, for example, a total length is 1,800 to 3,000 mm and an outer diameter is 0.03 mm to 0.46 mm.

The first layer 11 has a hollow cylindrical shape and contains a fluororesin having an adhesive functional group (adhesive resin 111), and a first colorant 112. The adhesive resin 111 is transparent to visible light. Thereby, colors, shapes, and the like of incorporated members (e.g. the first colorant 112) and members located on a back side can be visually recognized via this resin.

A polymer constituting the adhesive resin 111 has a fluorine-containing monomer unit such as a fluorine-containing ethylenic monomer unit.

Specific examples of a fluorine-containing ethylenic monomer include tetrafluoroethylene, vinylidene fluoride, chlorotrifluoroethylene, vinyl fluoride, hexafluoropropylene, hexafluoroisobutene, a perfluoro(alkylvinylether), a monomer represented by the following formula (1), and the like.

CH₂=CX¹(CF₂)ₙX² ... (1)

In the above formula (1), X¹ represents hydrogen atom or fluorine atom, X² represents hydrogen atom, fluorine atom, or chlorine atom, and n represents an integer of 1 to 10.

The polymer constituting the adhesive resin 111 may include a fluorine-free monomer unit such as a fluorine-free ethylenic monomer unit.

Examples of the fluorine-free ethylenic monomer include ethylene, propylene, 1-butene, 2-butene, vinyl chloride, vinylidene chloride, and the like.

The polymer constituting the adhesive resin 111 is preferably a tetrafluoroethylene-perfluoroalkylvinylether copolymer (PFA). This polymer makes it possible to improve adhesiveness of the entire fluororesin (adhesive resin 111) having an adhesive functional group. The tetrafluoroethylene-perfluoroalkylvinylether copolymer has a high melting point, good compatibility in dissolution with polytetrafluoroethylene (PTFE), and high mechanical properties.

The adhesive functional group in the adhesive resin 111 is not particularly limited as long as the adhesive functional group can react with a polar functional group of an adjacent material such as a resin or metal, or can cause an intermolecular interaction such as hydrogen bonding. The adhesive functional group may be present in at least one of a main-chain terminal and a side chain of the aforementioned fluororesin. The adhesive functional group preferably has a carbonyl group.

Examples of the adhesive functional group having the carbonyl group include a carbonyl group, a carbonate group, a halogenoformyl group, a formyl group, a carboxy group, a carbonyloxy group, an acid anhydride group, an isocyanate group, an amide group, an imide group, a urethane bond, a carbamoyl group, a carbamoyloxy group, a ureido group, an oxamoyl group, and the like.

Examples of a halogen atom constituting the aforementioned halogenoformyl group include fluorine atom, chlorine atom, and the like.

Above all, the adhesive functional group is preferably the carbonate group and/or the halogenoformyl group from the viewpoint of improving a reactivity (adhesiveness) with an adjacent material, and the like.

A production method for the adhesive resin 111 is not particularly limited, and a publicly known method can be used. Examples of the production method include a method in which a fluorine-containing ethylenic monomer, an adhesive functional group-containing ethylenic monomer, and, if necessary, a fluorine-free ethylenic monomer are blended in a predetermined ratio, and these components are copolymerized by a publicly known process, and the like.

Since the first layer 11 contains the fluororesin having an adhesive functional group (adhesive resin 111), the first layer 11 has an excellent reactivity between the adhesive functional group and the material constituting the core wire 31 (e.g. reactivity with a polar group such as a carboxy group or a hydroxyl group on the surface of the metal core wire 31), so that the first layer 11 and the core wire 31 can be strongly bonded to each other.

The first colorant 112 imparts color to the first layer 11. Examples of the materials constituting the first colorant 111 include inorganic pigments, organic pigments, and other pigments. Above all, inorganic pigments are preferable from the viewpoint of high heat resistance, discoloration resistance, and the like. Each of the materials may be used alone or in combination of two or more types.

Examples of the inorganic pigments include carbon black, mica, titanium dioxide, nickel titanium yellow, Prussian blue, Milori blue, cobalt blue, ultramarine, viridian, and the like.

The average particle diameter of the pigments is not particularly limited, but is preferably 0.02 µm to 5 µm, more preferably 0.1 µm to 1.0 µm. A content of the pigments is not particularly limited unless the effect of the disclosed embodiments is impaired, but is preferably 20% to 50% by mass, more preferably 30% to 40% by mass with respect to the content of the entire first layer 11.

The second layer 21 is arranged in contact with the outer peripheral surface of the first layer 11 and has a predetermined colored region R formed of a second colorant 212. The predetermined colored region R is formed on a part of the outer peripheral surface of the first layer 11.

For example, the second layer 21 may have a transparent resin 211 different from the fluororesin having the adhesive functional group (adhesive resin 111) so that the predetermined colored region R is disposed in the transparent resin 211, as illustrated in FIG. 1, FIG. 2A, and FIG. 2B. Thereby, the colors of the first colorant 112 and the second colorant 212 described below can be reliably recognized, and furthermore the predetermined colored region R can be prevented from peeling off from the first layer 11.

The second colorant 212 imparts a color to the second layer 21. The second colorant 212 has a color different from the color of the first colorant 112.

Examples of the material constituting the second colorant 212 include the same colorants as those described as the material constituting the first colorant 112 as examples, and the like.

The transparent resin 211 is different from the adhesive resin 111 described above, and is transparent to visible light (the colors of the first colorant 112 and second colorant 212 can be visually recognized via the resin 211).

For example, the transparent resin 211 may be at least one type of resin selected from a group consisting of polytetrafluoroethylene (PTFE), tetrafluoroethylene-perfluoroalkylvinylether copolymer (PFA), tetrafluoroethylene-hexafluoropropylene copolymer (FEP), nylon, and urethane. This resin makes it possible to enhance slidability of the outer peripheral surface of the second layer 21, so that, for example, the elongated medical device 1 can be smoothly operated when inserted into a body cavity.

Since the first layer 11 contains the fluororesin having an adhesive functional group (adhesive resin 111), the first layer 11 is excellent in reactivity between the adhesive functional group, and the second colorant 212 and the transparent resin 211 constituting the second layer 21 (e.g. reactivity with a polar group such as a carboxy group and a hydroxyl group on the surface of the second layer 21), so that the first layer 11 and the second layer 21 can be strongly bonded to each other.

Next, a usage mode of the guide wire 100 will be explained. Herein, a procedure for penetrating a distal end portion of the guide wire 100 into an occluded site in a body cavity will be explained.

First, the guide wire 100 is inserted from its distal end into a body cavity, and the distal end of the guide wire 100 is pushed forward to the occluded site while operating a part of the guide wire 100, which is exposed outside the body. An endoscope (not illustrated) is inserted together with the guide wire 100 to the vicinity of the occluded site, and a state of the guide wire 100 (e.g. a positional relationship between the distal end portion of the guide wire 100 and the occluded site) is observed.

In this process, the predetermined colored region R can be clearly recognized using the endoscope, because the second layer 21 formed on the guide wire 100 has the predetermined colored region R formed of the second colorant 212 having a color different from that of the first colorant 112. For example, when punctuating the guide wire 100 into the occluded site, an advancing degree of the guide wire 100 toward the occluded site, and the like can be reliably ascertained by observing the position of the colored region R.

After penetrating the guide wire 100 into the occluded site, various treatments may be performed by inserting a medical device such as a balloon catheter and a stent (not illustrated) over the guide wire 100, and transporting the medical device along the guide wire 100 to the perforated occluded site. After the treatments are completed, the guide wire 100, the endoscope, and the like are removed outside the body to complete the series of procedures.

As described above, since the elongated medical device 1 has the above configuration, the second layer 21 having the predetermined colored region R with a color different from that of the first layer 11 as a base can be reliably provided on the first layer 11. As a result, the position of the elongated medical device 1 can be reliably identified using the predetermined colored region R as a clue. The elongated medical device 1 further includes the core wire 31, so that the elongated medical device 1 can be desirably used as a guide wire.

### [Second Embodiment]

The second embodiment is intended to further enhance the adhesiveness between the core wire and the first layer containing the adhesive resin compared to the first embodiment.

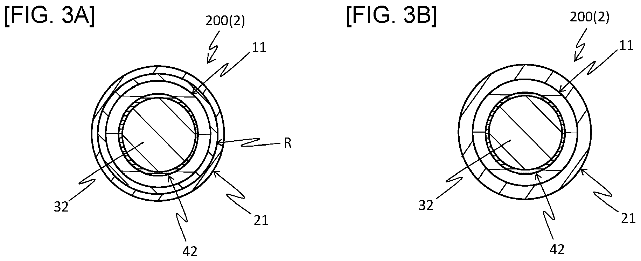

Each of FIG. 3A and FIG. 3B is a schematic transverse sectional view illustrating the second embodiment. FIG. 3A corresponds to a site cut along line IIA-IIA in FIG. 1, and FIG. 3B corresponds to a site cut along line IIB-IIB in FIG. 1. As illustrated in FIG. 3A and FIG. 3B, an elongated medical device 2 is schematically composed of a core wire 32, an intermediate layer 42, the first layer 11, and the second layer 21. In the second embodiment, as the elongated medical device 2, a guide wire 200 is described as an example. The guide wire 200 differs from the guide wire 100 in the first embodiment in that the guide wire 200 includes the core wire 32 and the intermediate layer 42. Since the configurations of the first layer 11 and the second layer 21 are the same as those in the first embodiment, the same portions are designated by the same reference numerals and detailed description thereof will be omitted. Configurations other than the configuration of guide wire 2 described below, and a usage mode of the guide wire 200 are the same as those in the first embodiment.

The core wire 32 is a metal member that serves as a core material for the elongated medical device 2. The core wire 32 is arranged in contact with the inner peripheral surface of the intermediate layer 42 described below.

The intermediate layer 42 is an adhesive layer having a hollow cylindrical shape and arranged in contact with the inner peripheral surface of the first layer 11. The intermediate layer 42 is specifically disposed e.g. between the first layer 11 and the core wire 32 and arranged in contact the inner peripheral surface of the first layer 11 and the outer peripheral surface of the core wire 32 over the entire length thereof along a longitudinal direction of the guide wire 200. For example, the intermediate layer 42 can be configured so as to have a uniform thickness over the entire longitudinal direction of the guide wire 200.

Examples of materials constituting the intermediate layer 42 include fluorine-free resins such as polysulfone, polyimide, polyetheretherketone, polyaryleneketone, polyphenylene sulfide, polyarylene sulfide, polyamideimide, polyetherimide, polyimidesulfone, polyarylsulfone, polyarylether sulfone, polyester, polyethersulfone, and epoxy resin, and the like.

Above all, polyamideimide and polyethersulfone are preferable. These materials make it possible to strongly bond the first layer 11 and the core wire 32 to each other via the intermediate layer 42. Each of the materials may be used alone or in combination of two or more types. The intermediate layer 42 may also contain materials other than those listed above.

As described above, since the guide wire 200 includes an adhesive intermediate layer 42, the first layer 11 and the core wire 32 can be reliably bonded to each other through the intermediate layer 42.

In the second embodiment, since the first layer 11 contains the first colorant 112 and the adhesive resin, the visibility of the guide wire 200 can be improved even when providing the intermediate layer 42 containing a resin with high adhesiveness with the core wire 32, and furthermore the adhesiveness between the core wire 32, and the intermediate layer 42 and the first layer 11 can be improved. In addition, the intermediate layer 42 need not contain any fluororesin, and the intermediate layer 42 can be made relatively thin.

### [Third Embodiment]

The third embodiment is intended to improve e.g. visibility in a catheter. The catheter also has a hollow metal layer to prevent crushing in a circumferential direction. The third embodiment is further intended to improve the adhesiveness between this metal layer and the resin layer that covers the metal layer.

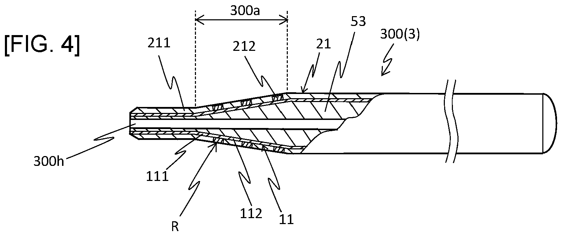

FIG. 4 is a schematic longitudinal sectional view illustrating the third embodiment. As illustrated in FIG. 4, an elongated medical device 3 is schematically composed of a metal layer 53, the first layer 11, and the second layer 21. In the third embodiment, as the elongated medical device 3, a dilator 300 (catheter) is described as an example. The elongated medical device 3 differs from the elongated medical device 1 in the first embodiment in that the elongated medical device 3 includes the hollow metal layer 53 instead of the core wire. Since the configurations of the first layer 11 and the second layer 21 are the same as those in the first embodiment, the same portions are designated by the same reference numerals and detailed description thereof will be omitted.

The metal layer 53 is a metal portion having a substantially hollow cylindrical shape and arranged in contact with the inner peripheral surface of the first layer 11. The metal layer 53 can be specifically configured e.g. as a hollow shaft having a lumen 300h.

Examples of the metal constituting the metal layer 53 include metal materials, e.g. stainless steel such as SUS304 and SUS316; superelastic alloys (nickel-titanium alloy); and the like.

Similarly to the first embodiment, the first layer 11 contains a fluororesin having an adhesive functional group (adhesive resin 111) and therefore has excellent reactivity between the adhesive functional group and the material constituting the metal layer 53 (e.g. reactivity with a polar group such as a carboxy group and a hydroxyl group on the surface of the metal layer 53), so that the first layer 11 and the metal layer 53 can be strongly bonded to each other.

Next, a usage mode of the elongated medical device 3 will be explained as an example. Herein, a procedure in which the elongated medical device 3 is a dilator 300 (catheter) and a constricted site formed in a body cavity such as a bile duct is dilated will be explained.

First, a guide wire (not illustrated) is inserted into the body cavity and pushed forward to an inner space of the constricted site. An endoscope (not illustrated) is inserted together with the guide wire to the vicinity of the treatment site. Subsequently, the proximal end of the guide wire is inserted into the lumen 300h of the hollow shaft of the dilator 300 from the distal end side of the dilator 300, and a dilation portion 300a of the hollow shaft is pushed forward to the constricted site.

In this process, since the second layer 21 formed on the dilator 300 has the predetermined colored region R formed of the second colorant 212 having a color different from that of the first colorant 112, the predetermined colored region R can be clearly recognized using the endoscope. For example, when dilating the constricted site by the dilation portion 300a of the dilator 300, an advancing degree of the dilation portion 300a of the dilator toward the constricted site can be reliably ascertained by observing the position of the colored region R in the dilation portion 300a.

After the dilatation of the constricted site using the dilator 300 is completed, the guide wire and the dilator 300 are removed outside the body to complete the series of procedures.

As described above, since the elongated medical device 3 has the above configuration, the second layer 21 having the predetermined colored region R with a color different from that of the first layer 11 as a base can be reliably provided on the first layer 11. As a result, the position of the elongated medical device 3 can be reliably identified using the predetermined colored region R as a clue. The elongated medical device 3 further includes the metal layer 53, so that the elongated medical device 3 can be desirably used as a catheter such as a dilator.

In the third embodiment, since the first layer 11 contains the first colorant, adhesiveness with the second layer and visibility can be improved. Furthermore, the first layer 11 is disposed between the metal layer 53 and the second layer 21 having the colored region to improve the adhesiveness between the metal layer 53 and the second layer 21.

### [Fourth Embodiment]

In the fourth embodiment, a resin tubular layer having a lumen on an inner peripheral side of the metal layer may be provided. The fourth embodiment is intended to further improve, compared to the third embodiment, adhesiveness between the metal layer, the tubular layer, and the resin layer on the outer periphery of the metal layer.

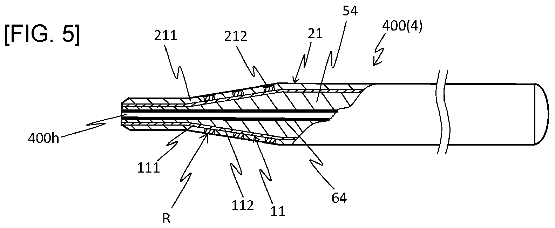

FIG. 5 is a schematic longitudinal sectional view illustrating the fourth embodiment. As illustrated in FIG. 5, an elongated medical device 4 is schematically composed of a sliding layer 64, a metal layer 54, the first layer 11, and the second layer 21. In the fourth embodiment, as the elongated medical device 4, a dilator 400 (catheter) is described as an example. The elongated medical device 4 differs from the elongated medical device 3 in the third embodiment in that the elongated medical device 4 includes the sliding layer 64 and the metal layer 54. Since the configurations of the first layer 11 and the second layer 21 are the same as those in the third embodiment, the same portions are designated by the same reference numerals and detailed description thereof will be omitted. Configurations other than that of the dilator 400 described below and a usage mode of the dilator 400 are the same as those in the third embodiment.

The sliding layer 64 is hollow cylindrical and disposed on the innermost side in the radial direction. Specifically, for example, the sliding layer 64 is disposed on the inner peripheral surface of the metal layer 54 and is arranged over the entire longitudinal direction of the dilator 400. For example, the sliding layer 64 can be configured such that a lumen 400h has a uniform inner diameter over the entire longitudinal direction of the dilator 400.

Examples of materials constituting the sliding layer 64 include resins such as polytetrafluoroethylene, ethylene-propylene fluoride, tetrafluoroethylene-perfluoroalkylvinylether copolymer, polychlorotrifluoroethylene, polyvinylidene fluoride, polyvinyl fluoride, tetrafluoroethylene-ethylene copolymer, and the like.

Above all, polytetrafluoroethylene (PTFE) and tetrafluoroethylene-perfluoroalkylvinylether copolymer are preferable, and polytetrafluoroethylene is more preferable. These materials make it possible to further improve the slidability.

The metal layer 54 has gaps (not illustrated) through which the material constituting the layers located on both sides of the metal layer 54 can penetrate. Specifically, for example, the metal layer 54 can be composed of braided bodies of strands. Thereby, the above materials can be fusion-bonded to each other via the gaps between the strands.

Examples of the metal constituting the metal layer 54 include the same metals as the metals constituting the metal layer 53 described above, and the like.

As described above, since the elongated medical device 4 has the aforementioned configuration, for example, it is possible to improve slidability of a medical device such as a guide wire that is inserted into the lumen 400h of the dilator 400 and comes into contact with the sliding layer 64, to smoothly proceed the procedure using the elongated medical device 4.

In the fourth embodiment, since the first layer 11 contains a first colorant, adhesiveness with the second layer and visibility can be improved. Furthermore, the adhesive resin of the first layer 11 melts and penetrates the gaps of the metal layer 54 (e.g. gaps between the strands of the braided body), so that the sliding layer 64 and the first layer 11 can be bonded to each other.

It should be noted that the present disclosure is not limited to the configurations of the aforementioned embodiments but defined by the scope of claims. The present disclosure is intended to include all modifications within the meaning and scope equivalent to those of the claims. A part of the configurations of the aforementioned embodiments may be deleted or replaced with another configuration, and other configurations may be added to the configurations of the aforementioned embodiments.

For example, in the first and second embodiments, the guide wires 100 and 200 including the core wires 31 and 32 and the intermediate layer 42 have been explained, and in the third and fourth embodiments, the dilators 300 and 400 (catheters) including the metal layers 53 and 54 and the sliding layer 64 have been explained. However, the elongated medical device only needs to include at least the first layer 11 and second layer 21 described above. The elongated medical device need not include at least any of the core wire, the intermediate layer, the metal layer, and the sliding layer. Also, the elongated medical device may include other core materials and layers (e.g. another base layer, intermediate layer, covering layer) together with or instead of the aforementioned core wire or the like.

### REFERENCE SIGNS LIST

1 to 4. Elongated medical device
100, 200. Guide wire
300, 400. Dilator
11. First layer
111. Adhesive resin
112. First colorant
21. Second layer
212. Second colorant
31, 32. Core wire
42. Intermediate layer
53, 54. Metal layer
64. Sliding layer
R. Colored region

## Claims

1. An elongated medical device comprising:
a first layer having a hollow cylindrical shape and containing a fluororesin having an adhesive functional group and a first colorant; and
a second layer arranged in contact with an outer peripheral surface of the first layer and having a predetermined colored region formed of a second colorant having a color different from a color of the first colorant; wherein
the predetermined colored region is formed on a part of the outer peripheral surface of the first layer.

2. The elongated medical device according to claim 1, wherein the predetermined colored region is disposed in a transparent resin different from the fluororesin having the adhesive functional group.

3. The elongated medical device according to claim 2, wherein the transparent resin is at least one selected from a group consisting of polytetrafluoroethylene, tetrafluoroethylene-perfluoroalkylvinyl ether copolymer, tetrafluoroethylene-hexafluoropropylene copolymer, nylon, and urethane.

4. The elongated medical device according to any one of claims 1 to 3, further comprising a metal core wire arranged in contact with an inner peripheral surface of the first layer.

5. The elongated medical device according to any one of claims 1 to 3, further comprising:
an adhesive intermediate layer having a hollow cylindrical shape and arranged in contact with the inner peripheral surface of the first layer; and
a metal core wire arranged in contact with an inner peripheral surface of the intermediate layer.

6. The elongated medical device according to any one of claims 1 to 3, further comprising a metal layer having a substantially hollow cylindrical shape and arranged in contact with the inner peripheral surface of the first layer.

7. The elongated medical device according to any one of claims 1 to 3, and 6, further comprising a sliding layer having a hollow cylindrical shape and disposed on an innermost side in a radial direction.
